(19) Europäisches Patentamt
European Patent Office
Office européen des brevets

(11) Publication number: **0 010 523**
**B1**

(12) **EUROPEAN PATENT SPECIFICATION**

(45) Date of publication of patent specification: **22.09.82**

(51) Int. Cl.³: **C 11 D 3/34,** C 07 C 149/18, B 01 F 17/00

(21) Application number: **79810129.1**

(22) Date of filing: **15.10.79**

(54) **Perfluoroalkylalkylenemercapto group containing non-ionic surfactants, process for their manufacture and their use.**

(30) Priority: **20.10.78 US 953296**

(43) Date of publication of application:
**30.04.80 Bulletin 80/9**

(45) Publication of the grant of the patent:
**22.09.82 Bulletin 82/38**

(84) Designated Contracting States:
**BE CH DE FR GB IT NL**

(56) References cited:
**DE - A - 2 342 888**
**DE - A1 - 2 749 330**
**DE - A1 - 2 758 013**

The file contains technical information submitted after the application was filed and not included in this specification

(73) Proprietor: **CIBA-GEIGY AG**
**Patentabteilung Postfach**
**CH-4002 Basel (CH)**

(72) Inventor: **Cooke, Thomas W.**
**R.D. 3, Red Hill Road**
**Mahopac, New York 10541 (US)**

**EP 0 010 523 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Perfluoroalkylalkylenemercapto group containing non-ionic surfactants, process for their manufacture and their use

The present invention relates to novel fluorinated non-ionic surfactants.

From DE—A1—27 58 013 it is known to improve the surface properties of solutions of fluorinated surfactants, e.g. of the formula $(R_f)_nA_mQ$ by employing a fluorinated synergist and optionally magnesium salts. $R_f$ in the formula is a perfluoroalkyl group, A is a bridging member, Q is a water solubilizing group, e.g. an anionic, cationic, non-ionic or amphoteric group, n is 1 or 2 and m is zero to 2.

As non-ionic groups radicals of polyethylene oxide/polypropylene oxide polyols are mentioned. The bridging member comprises alkylene or arylene moieties and is quite different from the bridging member of the inventive compounds shown in the following formula (1). These new compounds of the inventive type are not suggested by the reference cited.

One object of the present invention is the provision of novel fluorinated non-ionic surfactants of the general formula

$$R_f—A—SCH_2—\underset{\underset{OH}{|}}{CH}—CH_2O—(—CH_2\underset{\underset{R_1}{|}}{C}HO—)_n—R_2 \qquad (1)$$

wherein $R_f$ is perfluoroalkyl of 4 to 20 carbon atoms, or perfluoroalkoxyperfluoroalkyl of 4 to 20 carbon atoms, A is straight or branched chain alkylene of 1 to 10 carbon atoms which is unsubstituted or is substituted by halogen, preferably by chloro or fluoro, n is 1 to 50, $R_1$ is hydrogen or alkyl of 1 to 4 carbon atoms and $R_2$ is hydrogen or alkyl of 1 to 4 carbon atoms.

Preferably, $R_f$ is perfluoroalkyl of 4 to 16, preferably 4 to 14 carbon atoms or said perfluoroalkyl substituted by perfluoroalkoxy of 2 to 4 carbon atoms, A is unsubstituted alkylene of 1 to 4 carbon atoms, preferably ethylene, n is 5 to 25, $R_1$ is hydrogen or methyl, $R_2$ is hydrogen or alkyl of 1 to 4 carbon atoms, most preferably methyl and n is 5 to 50.

Where n is less than 5, the resulting compounds have little water solubility. However, such compounds are useful as surfactants in non-aqueous systems, as mold release agents in the plastics and silicone industry, as well as grease and oil repellants for paper. Those compounds wherein $R_1$ is predominantly lower alkyl are likewise characterized by their limited water solubility and likewise possess utility as surfactants in non aqueous systems, as mold release agents and as grease and oil repellants for paper and the like.

Preferred compounds are those wherein $R_1$ is at least predominantely hydrogen, $R_2$ is alkyl of 1 to 4 carbon atoms, and n is 5 to 50, preferably 5 to 25. Such compounds are essentially water soluble.

Such water soluble compounds are valuable aqueous surfactants and are useful industrial wetting agents. Thus, they are useful wetting agents for biocides, cleaning compositions and detergents, as rinse-aids for cars, dishes and the like, as emulsifiers, and as levelling agents in the dye and pigment industry.

The compounds of the instant invention are easily prepared by any one of a number of routes.

The compounds can be prepared conveniently by reacting an alkoxylated compound of the formula

$$R_2(O\underset{\underset{R_1}{|}}{C}HCH_2)_nOH \qquad (2)$$

with epichlorohydrin in bulk or in a common dry and aprotic solvent, including ketones, such as acetone or methyl ethlyl ketone; ethers, such as diethylether, ethyleneglycol-dimethylether or tetrahydrofuran; esters such as ethyl acetate or methyl ethyleneglycol acetate; and amides, such as dimethylformamide or N-methyl pyrrolidone. A Lewis acid catalyst, such as boron trifluoride (usually in the form of the diethyl ether complex thereof) or aluminium chloride, is used to promote the formation of the halohydrin intermediate. If the reaction is run in the absence of a solvent, it is advantageously run at a temperature above the melting point of the alkoxylated compound with boron trifluoride-etherate. The reaction is ordinarily exothermic. It is generally not necessary to isolate the halohydrin intermediate, and the next reaction can be carried out in the same vessel. The halohydrin intermediate has the formula

$$ClCH_2\underset{\underset{OH}{|}}{C}HCH_2O(CH_2\underset{\underset{R_1}{|}}{C}HO)_nR_2 \qquad (3)$$

In the second step, the perfluoroalkylalkylene mercaptan ($R_f$—A—SH) (US-Patent Specification 3,172,910) is added to the halohydrin intermediate, followed by the addition of an equivalent amount

of base as an acid acceptor. This step may advantageously be carried out in bulk or in the presence of aprotic or protic solvents. As aprotic solvents, there may be used those recited above for the first step. Suitable protic solvents include water, and alcohols such as methanol, ethanol, isopropanol, tert. butyl alcohol, hexyleneglycol, ethyleneglycol and ethyleneglycol monoethylether. Suitable bases include sodium hydroxide, potassium hydroxide, pyridine, lutidine and triethylamine. Conveniently, a solvent is selected which dissolves the reactants and desired product, but not the by-product salt, so that the salt will precipitate out as the reaction proceeds and can then be removed by conventional techniques, such as filtration. The fluorochemical surfactant product may be left in solution or evaporated to remove the solvent. The reaction is preferably carried out between about 30 and 80°C, under a nitrogen atmosphere, with stirring. There is generally an exotherm as the base is added to the reaction mixture.

Alternatively, the surfactants of the instant invention may be prepared by first reacting equimolar amounts of perfluoroalkylalkylene mercaptan, $R_f$—A—SH, and epichlorohydrin by placing them in a reaction vessel and stirring the reactants in the presence of an inert solvent, such as methyl ethyleneglycol acetate. Then a stoichiometric amount of a base, such as 50% aqueous sodium hydroxide, is slowly added to the reaction mixture. The resulting reaction is exothermic and the reaction temperature is advantageously maintained between about 20 to 60°C with stirring for 1 to 3 hours. The resulting epoxide intermediate has the formula

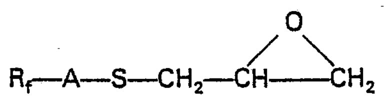

(4)

The by-product salt is removed from the epoxide intermediate and any residual water is removed by vacuum distillation.

The epoxide intermediate is then reacted with an equimolar amount, or a slight excess, of an alkoxylated compound of the formula (2), which preferably is a (poly)ethyleneglycol ($R_2$=$R_1$=H), a (poly)-propyleneglycol ($R_1$=$CH_3$, $R_2$=H) or an ethoxylated (propoxylated) lower alcohol ($R_2$=alkyl of 1 to 4 carbon atoms). These compounds are well known in the art.

This reaction is advantageously conducted under substantially anhydrous conditions in the presence of a catalyst, such as $BF_3 \cdot$ etherate, at a temperature of about 30 to 60°C in the presence or absence of an inert solvent, such as methyl ethyleneglycol acetate. In one technique, the catalyst is dispersed in a mixture of the alkoxylated compound and the solvent, and slowly added to the epoxide intermediate with stirring. Preferably, this reaction is carried out under a nitrogen blanket.

The product fluorochemical surfactant may be left in solution and used as such, or may be evaporated or distilled under vacuum to remove the solvent.

The fluorochemical surfactants of this invention are distinguished by their increased surface active properties vis-a-vis their related hydrocarbon surfactant analogs (cf. US 3,758,595). As a result, they improve or impart properties such as wetting, penetration, spreading, leveling, foam stability, flow properties, dispersion properties and oil and water repellency.

In the following Examples all parts are by weight unless otherwise specified.

Example 1:

32.56 g (0.3520 moles) of epichlorohydrin was slowly added to a mixture of 140.0 g (0.4000 moles) Carbowax 350 (methoxypolyethoxy ethanol, MW≅350) and 1.60 g $BF_3 \cdot$ diethyl etherate (47% $BF_3$) at a rate which maintained the exothermic reaction at 50 to 60°C. After stirring for an additional 1/2 hour at 50 to 60°C, 355.4 g of anhydrous isopropyl alcohol and 148.8 g (0.3200 moles) of perfluoroalkylethyl-mercaptan *($R_fCH_2CH_2SH$, MW≅465) were added to the reaction vessel and stirred at ~50°C. Then 28.67 g (0.3584 moles) of 50% aq. sodium hydroxide was slowly added to the reaction mixture at a rate which maintained the exothermic reaction at 50 to 60°C. A white precipitate formed (sodium chloride by-product) during the addition. The system was stirred for an additional 1 hour at 50 to 55°C. After cooling to ~25°C, the resulting product mixture was filtered to remove the NaCl. 647 g of an amber solution were collected (at 94.3% yield of solution), containing the compound with the structure:

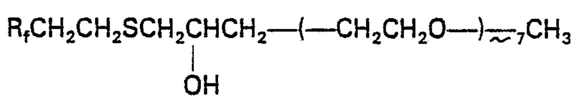

*$R_f$=0.9% $C_4F_9$, 32.9% $C_6F_{13}$, 37.5% $C_8F_{17}$, 22.9% $C_{10}F_{21}$, 5.3% $C_{12}F_{15}$

This product was slightly soluble in water and soluble in toluene. A portion of the solution was evaporated to solids (43.5% solids found) leaving an amber oil. 33.0% F found (32.1% F calc.)

Example 2

In the same manner as Example 1, 24.42 g (0.2640 moles) of epichlorohydrin, 225.0 g (0.3000 moles) of Carbowax 750 (methoxypolyethoxy ethanol, MW≅750), 1.60 g $BF_3 \cdot$ diethyl etherate, 412.2

g anhydrous isopropyl alcohol, 111.6 g (0.2400 moles) of perfluoroalkylethyl mercaptan (same $R_f$-distribution as in Example 1), and 21.50 g (0.2688 moles) of 50% aq. sodium hydroxide were utilized to prepare 742 g (95.0% yield) of a solution containing the compound:

$$R_fCH_2CH_2SCH_2\underset{\displaystyle OH}{\underset{|}{C}}HCH_2O(CH_2CH_2O)_{\sim 16}CH_3$$

The product was an amber solution (43.2% solids found) which was soluble in water and toluene. 21.3% F found in solids (21.1% F calc.).

Measured surface properties in deionized water at 25°C

| Compound from | Measurement | Level of Actives 0.1% | 0.01% | 0.003% | 0.001% |
|---|---|---|---|---|---|
| Example 1 | surface tension ($\cdot 10^{-5}$ N/cm) | 21.0 | 22.3 | 32.9 | — |
| | interfacial tension ($\cdot 10^{-5}$ N/cm) | 12.1 | 17.2 | — | 24.3 |
| Example 2 | surface tension ($\cdot 10^{-5}$ N/cm) | 24.1 | 26.1 | 31.5 | 32.8 |
| | interfacial tension ($\cdot 10^{-5}$ N/cm) | 10.9 | 12.8 | — | 18.7 |

note: interfacial tension measured against cyclohexane.

Examples 3 to 8:

Using the procedure of Example 1, compounds of the general structure

$$R_fCH_2CH_2S{-}CH_2\underset{\displaystyle OH}{\underset{|}{C}}HCH_2O{-}({-}CH_2CH_2O{-})_{\overline{n}}R$$

were prepared from the hydroxy compounds listed below, where "n" and "R" are defined in the table, and $R_f \cong 1.7\%\ C_4F_9$, 32.9% $C_6F_{13}$, 36.4% $C_8F_{17}$, 21.8% $C_{10}F_{21}$, 5.4% $C_{12}F_{25}$, 0.6% $C_{14}F_{29}$.

| Example | Non-ionic Moiety | Value of "n" | Structure of "R" | Surface Tension ($\cdot 10^{-5}$ N/cm), in water, at 0.1% actives | /at 0.1% F |
|---|---|---|---|---|---|
| 3 | ethyleneglycolmonomethylester | 1 | $-CH_3$ | not soluble | not soluble |
| 4 | methoxytriglycol | 3 | $-CH_3$ | slightly sol. | slightly sol. |
| 5 | methoxypolyethoxyethanol, MW=550 (Carbowax 550) | ~12 | $-CH_3$ | 22.0 | 22.2 |
| 6 | Pluronic L-42 | ~9 | $-(-i\text{-}PrO)_{\sim 21}H$ | 22.7 | 22.6 |
| 7 | Pluronic P-65 | ~38 | $-(-i\text{-}PrO)_{\sim 30}H$ | 30.0 | 29.3 |
| 8 | ethyleneglycolmonobutylester | 1 | $-C_4H_9$ | not soluble | not soluble |

Example 9

By the process of Example 1, 4.1 g (0.044 moles) of epichlorohydrin, 95.0 g (0.05 moles) of Carbowax 2000 (methoxypolyethylene glycol, MW≅1900), 0.3 g $BF_3\cdot$ether complex, 146.3 g anhydrous isopropyl alcohol, 23.6 g (0.04 moles) of perfluoroalkylethyl mercaptan (MW≅588.8, see $R_f$-distribution below), and 3.6 g (0.0448 moles) of 50% sodium hydroxide were used to prepare the compound:

$$R_fCH_2CH_2SCH_2\underset{\displaystyle OH}{\underset{|}{C}}HCH_2O{-}({-}CH_2CH_2O{-})_{\sim 42}CH_3$$

where

$R_f$=0.5% $C_6F_{13}$, 24.1% $C_8F_{17}$, 48.1% $C_{10}F_{21}$, 21.7% $C_{12}F_{25}$, 2.8% $C_{14}F_{29}$.

234.6 g of a thick white suspension (at room temperature) were obtained at 42.9% solids, found. The solids were a white hard wax.

Surface Tension Results: 31.5 ($\cdot 10^{-5}$ N/cm) 0.1% actives
48.1 ($\cdot 10^{-5}$ N/cm) 0.01% actives
61.3 ($\cdot 10^{-5}$ N/cm) 0.001% actives.

Example 10

Also by the process described in Example 1, 6.48 g (0.07 moles) of epichlorohydrin, 5.33 g (0.07 moles) of 2-methoxyethanol, 0.1 g of $BF_3 \cdot$ ether complex, 5.6 g of anhydrous isopropyl alcohol, 33.72 g (0.07 moles) of perfluorooctylethyl mercaptan ($C_8F_{17}CH_2CH_2SH$), and 5.60 g of 50% sodium hydroxide were used to prepare 37.4 g of isolated solids (87% yield) of the following compound:

$$C_8F_{17}CH_2CH_2SCH_2\underset{\displaystyle OH}{\underset{|}{C}}HCH_2OCH_2CH_2OCH_3$$

| | |
|---|---|
| calculated: | 31.38% C 2.80% H 52.74% F |
| found: | 30.43% C 2.51% H 52.99% F |

Example 11

By the process of Example 1, 2.31 g (0.025 moles) epichlorohydrin, 18.75 g (0.025 moles) of Carbowax MPEG 750 (Union Carbide Corp.), 0.1 g $BF_3 \cdot$ ether complex, 35.22 g anhydrous isopropyl alcohol, 8.65 g (0.025 moles) of $(CF_3)_3CFOCF_2CF_2CH_2CH_2SH$, and 2.00 g (0.25 moles) of 50% sodium hydroxide were used to prepare the compound:

$$(CF_3)_2CFOCF_2CF_2CH_2CH_2SCH_2\underset{\displaystyle OH}{\underset{|}{C}}HCH_2O—(—CH_2CH_2O—)_{\approx 16}CH_3$$

The product (evaporated to solids) was a yellow wax.

Surface Tension=18.0 ($\cdot 10^{-5}$ N/cm at 0.1% in deionized water)

| | |
|---|---|
| Calculated: | 45.3% C 6.8% H 18.4% F |
| Found: | 45.5% C 7.1% H 15.6% F |

Examples 12 to 18

The following compounds were made in the same manner as Example 1, with the exception that ethyleneglycol monoethyl ether was used in the second step of the reaction as the solvent. The amount of the solvent used was twice the amount of solids present.

$$R_fCH_2CH_2SCH_2\underset{\displaystyle OH}{\underset{|}{C}}HCH_2O—(—CH_2CH_2O—)_{\overline{n}}R$$

In the Table below, the $R_f$-distribution of

$A_1$ is 33.0% $C_6F_{13}$, 37.5% $C_8F_{17}$, 23.0% $C_{10}F_{21}$, 5.3% $C_{12}F_{25}$ and 0.2% $C_{14}F_{29}$ (balance=inerts);
$B_1$ is 5.5% $C_6F_{13}$, 40.8% $C_8F_{17}$, 39.2% $C_{10}F_{21}$, 11.0% $C_{12}F_{25}$ and 1.1% $C_{14}F_{29}$ (balance=inerts); and
$C_1$ is 1.0% $C_6F_{13}$, 15.9% $C_8F_{17}$, 59.0% $C_{10}F_{21}$, 17.4% $C_{12}F_{25}$ and 2.2% $C_{14}F_{29}$ (balance=inerts).

| Examples | Prepared from | n | R | $R_f$ | Surface Tension Deionized Water ($\cdot 10^{-5}$ N/cm) at 0.1% | 0.01% | 0.001% | Appearance of Dilution in Water 1—2% solids |
|---|---|---|---|---|---|---|---|---|
| 12 | Carbowax 350 | 7 | $CH_3$ | $A_1$ | 20.0 | 21.8 | 37.1 | hazy |
| 13 | Carbowax 350 | 7 | $CH_3$ | $A_1$ | 19.8 | 22.1 | 36.1 | hazy |
| 14 | Carbowax 550 | 12 | $CH_3$ | $A_1$ | 22.3 | 24.1 | 39.7 | clear |
| 15 | Carbowax 350/550 (50/50 blend) | 7—12 | $CH_3$ | $A_1$ | 21.6 | 23.0 | 40.0 | clear |
| 16 | Carbowax 750 | 16 | $CH_3$ | $A_1$ | 24.0 | 28.9 | 44.7 | clear |
| 17 | Carbowax 750 | 16 | $CH_3$ | $B_1$ | 24.5 | 29.8 | 44.2 | slightly hazy |
| 18 | Carbowax 750 | 16 | $CH_3$ | $C_1$ | 24.0 | 30.0 | 54.6 | hazy |

## Claims

1. A fluorochemical surfactant of the formula

$$R_f—A—S—CH_2\underset{\displaystyle OH}{\underset{|}{C}}HCH_2O—(—CH_2\underset{\displaystyle R_1}{\underset{|}{C}}HO—)_{\overline{n}}R_2$$

wherein $R_f$ is perfluoroalkyl of 4 to 20 carbon atoms or perfluoroalkoxyperfluoroalkyl of 4 to 20 carbon atoms, A is straight or branched chain alkylene of 1 to 10 carbon atoms which is unsubstituted or substituted by halogen, n is 1 to 50, $R_1$ is hydrogen or alkyl of 1 to 4 carbon atoms, and $R_2$ is hydrogen or alkyl of 1 to 4 carbon atoms.

2. A fluorochemical surfactant according to Claim 1, wherein $R_f$ is perfluoroalkyl of 4 to 16 carbon atoms, or said perfluoroalkyl substituted by perfluoroalkoxy of 2 to 4 carbon atoms, A is unsubstituted alkylene of 1 to 4 carbon atoms, n is 5 to 50, $R_1$ is hydrogen or methyl, and $R_2$ is hydrogen or alkyl of 1 to 4 carbon atoms.

3. A fluorochemical surfactant according to Claim 2, wherein $R_1$ is hydrogen and $R_2$ is alkyl of 1 to 4 carbon atoms.

4. A fluorochemical surfactant according to Claim 3, wherein n is 5 to 25.

5. A fluorochemical surfactant according to Claim 2, wherein $R_2$ is alkyl of 1 to 4 carbon atoms and A is ethylene.

6. A fluorochemical surfactant according to Claim 5, wherein $R_2$ is methyl and A is ethylene.

7. A fluorochemical surfactant according to Claim 2, wherein $R_2$ is methyl and $R_1$ is hydrogen.

8. Process for the manufacture of the compounds according to Claim 1 which comprises reacting an alkoxylated compound of the formula

$$R_2(O\underset{\displaystyle R_1}{\underset{|}{CH}}CH_2)_nOH$$

wherein $R_1$ is hydrogen or alkyl of 1 to 4 carbon atoms, $R_2$ is hydrogen or alkyl of 1 to 4 carbon atoms and n is 1 to 50, with epichlorohydrin in bulk or in a dry and aprotic solvent in the presence of a Lewis acid, and then reacting this intermediate with a perfluoroalkylalkylene mercaptan of the formula

$$R_f—A—SH$$

wherein $R_f$ is perfluoroalkyl of 4 to 20 carbon atoms or perfluoroalkoxyperfluoroalkyl of 4 to 20 carbon atoms and A is a straight or branched chain alkylene of 1 to 10 carbon atoms which is unsubstituted or substituted by halogen, in the presence of a base as acid acceptor.

9. Process for the manufacture of the compounds according to Claim 1 which comprises reacting a perfluoroalkylalkylene mercaptan of the formula

$$R_f—A—SH$$

wherein $R_f$ is perfluoroalkyl of 4 to 20 carbon atoms or perfluoroalkoxyperfluoroalkyl of 4 to 20 carbon atoms and A is a straight or branched chain alkylene of 1 to 10 carbon atoms which is unsubstituted or substituted by halogen, with epichlorohydrin in an inert solvent and in the presence of a base, and then reacting the obtained epoxide intermediate with an alkoxylated compound of the formula

$$R_2(O\underset{\displaystyle R_1}{\underset{|}{CH}}CH_2)_nOH$$

wherein $R_1$ is hydrogen or alkyl of 1 to 4 carbon atoms, $R_2$ is hydrogen or alkyl of 1 to 4 carbon atoms and n is 1 to 50, under substantially anhydrous conditions in the presence or absence of an inert solvent and in the presence of a Lewis acid.

10. Use of the compounds according to Claim 1 as emulsifiers or wetting and levelling agents.

**Patentansprüche**

1. Fluorierte Tenside der Formel

$$R_f—A—S—CH_2—\underset{\displaystyle OH}{\underset{|}{CH}}—CH_2—O—(—CH_2\underset{\displaystyle R_1}{\underset{|}{C}}HO—)_{\overline{n}}R_2$$

worin $R_f$ Perfluoralkyl oder Perfluoralkoxyperfluoralkyl mit 4 bis 20 Kohlenstoffatomen, A geradkettiges oder verzweigtes, gegebenenfalls mit Halogen substituiertes Alkylen mit 1 bis 10 Kohlenstoffatomen, n 1 bis 50 ist, und $R_1$ und $R_2$ Wasserstoff oder Alkyl mit 1 bis 4 Kohlenstoffatomen sind.

2. Fluorierte Tenside gemäss Anspruch 1, worin $R_f$ gegebenenfalls mit Perfluoralkoxy mit 2 bis 4 Kohlenstoffatomen substituiertes Perfluoralkyl mit 4 bis 16 Kohlenstoffatomen, A unsubstituiertes Alkylen mit 1 bis 4 Kohlenstoffatomen, n 5 bis 50, $R_1$ Wasserstoff oder Methyl und $R_2$ Wasserstoff oder Alkyl mit 1 bis 4 Kohlenstoffatomen ist.

3. Fluorierte Tenside gemäss Anspruch 2, worin $R_1$ Wasserstoff und $R_2$ Alkyl mit 1 bis 4 Kohlenstoffatomen ist.

4. Fluorierte Tenside gemäss Anspruch 3, worin n 5 bis 25 ist.

5. Fluorierte Tenside gemäss Anspruch 2, worin $R_2$ Alkyl mit 1 bis 4 Kohlenstoffatomen und A Aethylen ist.

6. Fluorierte Tenside gemäss Anspruch 5, worin $R_2$ Methyl und A Aethylen ist.

7. Fluorierte Tenside gemäss Anspruch 2, worin $R_2$ Methyl und $R_1$ Wasserstoff ist.

8. Verfahren zur Herstellung von Verbindungen gemäss Anspruch 1, dadurch gekennzeichnet, dass man eine alkoxylierte Verbindung der Formel

$$R_2(O\underset{\displaystyle R_1}{\underset{|}{C}}HCH_2)_n{-}OH$$

worin $R_1$ und $R_2$ Wasserstoff oder Alkyl mit 1 bis 4 Kohlenstoffatomen sind und n 1 bis 50 ist, mit Epichlorhydrin, gegebenenfalls gelöst in einem trockenen und aprotischen Lösungsmittel, in Gegenwart einer Lewis-Säure umsetzt und das erhaltene Zwischenprodukt mit einem Perfluoralkylalkylenmercaptan der Formel

$$R_f{-}A{-}SH$$

worin $R_5$ Perfluoralkyl oder Perfluoralkoxyperfluoralkyl mit 4 bis 20 Kohlenstoffatomen und A geradkettiges oder verzweigtes, gegebenenfalls mit Halogen substituiertes Alkylen mit 1 bis 10 Kohlenstoffatomen ist, in Gegenwart einer Base als Säureakzeptor umsetzt.

9. Verfahren zur Herstellung von Verbindungen gemäss Anspruch 1, dadurch gekennzeichnet, dass man ein Perfluoralkylalkylenmercaptan der Formel

$$R_f{-}A{-}SH$$

worin $R_f$ Perfluoralkyl oder Perfluoralkoxyperfluoralkyl mit 4 bis 20 Kohlenstoffatomen und A gegebenenfalls mit Halogen substituiertes geradkettiges oder verzweigtes Alkylen mit 1 bis 10 Kohlenstoffatomen ist, mit Epichlorhydrin in einem inerten Lösungsmittel in Gegenwart einer Base umsetzt und das erhaltene Epoxy-Zwischenprodukt mit einer alkoxylierten Verbindung der Formel

$$R_2(O\underset{\displaystyle R_1}{\underset{|}{C}}HCH_2)_n{-}OH$$

worin $R_1$ und $R_2$ Wasserstoff oder Alkyl mit 1 bis 4 Kohlenstoffatomen sind und n 1 bis 50 ist, gegebenenfalls in einem inerten Lösungsmittel in Gegenwart einer Lewis-Säure umsetzt, wobei das Reaktionsmedium im wesentlichen wasserfrei ist.

10. Verwendung der Verbindungen gemäss Anspruch 1 als Emulgatoren, Netz- oder Egalisiermittel.

**Revendications**

1. Composé surfactif fluorochimique de formule:

$$R_f{-}A{-}S{-}CH_2\underset{\displaystyle OH}{\underset{|}{C}}HCH_2O{-}({-}CH_2\underset{\displaystyle R_1}{\underset{|}{C}}HO{-}){-}_nR_2$$

dans laquelle $R_f$ est un groupe perfluoroalkyle ayant 4 à 20 atomes de carbone ou un groupe perfluoroalcoxyperfluoroalkyle ayant 4 à 20 atomes de carbone; A est un groupe alkylène à chaîne droite ou ramifiée ayant 1 à 10 atomes de carbone qui est non substitué ou bien est substitué par de l'halogène; n vaut 1 à 50; $R_1$ est de l'hydrogène ou un groupe alkyle ayant 1 à 4 atomes de carbone, et $R_2$ est de l'hydrogène ou un groupe alkyle ayant 1 à 4 atomes de carbone.

2. Composé surfactif fluorochimique selon la revendication 1, dans lequel $R_f$ est un groupe perfluoroalkyle ayant 4 à 16 atomes de carbone, ou ledit groupe perfluoroalkyle substitué par un groupe perfluoroalcoxy ayant 2 à 4 atomes de carbone; A est un groupe alkylène non substitué ayant 1 à 4 atomes de carbone; n vaut 5 à 50; $R_1$ est de l'hydrogène ou le groupe méthyle, et $R_2$ est de l'hydrogène ou un groupe alkyle ayant 1 à 4 atomes de carbone.

3. Composé surfactif fluorochimique selon la revendication 2, dans lequel $R_1$ est de l'hydrogène, et $R_2$ est un groupe alkyle ayant 1 à 4 atomes de carbone.

4. Composé surfactif fluorochimique selon la revendication 3, dans lequel n vaut 5 à 25.

5. Composé surfactif fluorochimique selon la revendication 2, dans lequel $R_2$ est un groupe alkyle ayant 1 à 4 atomes de carbone, et A est une groupe éthylène.

6. Composé surfactif fluorochimique selon la revendication 5, dans lequel $R_2$ est le groupe méthyle et A est un groupe éthylène.

7. Composé surfactif fluorochimique selon la revendication 2, dans lequel $R_2$ est le groupe méthyle et $R_1$ est de l'hydrogène.

8. Procédé pour la fabrication des composés selon la revendication 1, qui comprend la réaction d'un composé alcoxylé de formule:

$$R_2(O\underset{\underset{R_1}{|}}{C}HCH_2)_nOH$$

dans lequelle $R_1$ est de l'hydrogène ou un groupe alkyle ayant 1 à 4 atomes de carbone; $R_2$ est de l'hydrogène ou un groupe alkyle ayant 1 à 4 atomes de carbone, et n vaut 1 à 50, avec l'épichlorhydrine, en masse ou dans un solvant sec et aprotique, en présence d'un acide de Lewis, puis la réaction de cet intermédiaire avec un perfluoroalkylalkylènemercaptan de formule:

$$R_f\text{—}A\text{—}SH$$

dans laquelle $R_f$ est un groupe perfluoroalkyle de 4 à 20 atomes de carbone ou un groupe perfluoroalcoxyperfluoroalkyle de 4 à 20 atomes de carbone, et A est un groupe alkylène à chaîne droite ou ramifiée ayant 1 à 10 atomes de carbone qui est non substitué ou substitué par de l'halogène, en présence d'une base comme accepteur d'acide.

9. Procédé pour la fabrication des composés selon la revendication 1, qui comprend la réaction d'un perfluoroalkylalkylènemercaptan de formule:

$$R_f\text{—}A\text{—}SH$$

dans laquelle $R_f$ est un groupe perfluoroalkyle ayant 4 à 20 atomes de carbone ou un groupe perfluoroalcoxyperfluoroalkyle ayant 4 à 20 atomes de carbone. et A est un groupe alkylène à chaîne droite ou ramifiée ayant 1 à 10 atomes de carbone qui est non substitué ou substitué par de l'halogène, avec de l'épichlorhydrine dans un solvant inerte et en présence d'une base, puis la réaction de l'intermédiaire époxyde obtenu avec un composé alcoxylé de formule:

$$R_2(O\underset{\underset{R_1}{|}}{C}HCH_2)_nOH$$

dans laquelle $R_1$ est de l'hydrogène ou un groupe alkyle ayant 1 à 4 atomes de carbone; $R_2$ est de l'hydrogène ou un groupe alkyle ayant 1 à 4 atomes de carbone, et n vaut 1 à 50, dans des conditions sensiblement anhydres, en présence ou en l'absence d'un solvant inerte et en présence d'un acide de Lewis.

10. Utilisation des composés selon la revendication 1 comme émulsifiants ou agents mouillants et agents d'unisson.